# EUROPÄISCHE PATENTSCHRIFT

(11) **EP 2 704 698 B1**
(45) Veröffentlichungstag und Bekanntmachung des Hinweises auf die Patenterteilung: **02.10.2019**
(21) Anmeldenummer: 12719667.3
(22) Anmeldetag: 02.05.2012
(51) Int. Cl.: A61K 9/28, A61K 9/48, A61K 9/16, A61K 9/20

(54) **ARZNEIFORM ZUR GEZIELTEN FREIGABE VON WIRKSTOFFEN**
DOSAGE FORM FOR THE CONTROLLED RELEASE OF ACTIVE INGREDIENTS
FORME GALÉNIQUE POUR LIBÉRATION CIBLÉE DE PRINCIPES ACTIFS

(30) Priorität: 05.05.2011 DE 102011075354; 05.05.2011 DE 102011075356
(43) Veröffentlichungstag der Anmeldung: 12.03.2014
(73) Patentinhaber: Hennig Arzneimittel GmbH&Co. Kg, 65439 Flörsheim am Main (DE)
(72) Erfinder: FRANCAS, Gernot, 67551 Worms (DE); PRZYKLENK, Karl-Heinz, 64521 Groß Gerau (DE)
(74) Vertreter: Fuchs Patentanwälte Partnerschaft mbB
(86) Internationale Anmeldenummer: PCT/EP2012/057996
(87) Internationale Veröffentlichungsnummer: WO 2012/150246

(56) Entgegenhaltungen:
- WO-A2-2006/078811
- WO-A2-2007/083323
- US-A1- 2002 054 911
- US-A1- 2003 049 318
- US-A1- 2003 068 366
- US-A1- 2003 077 297
- US-A1- 2007 184 110
- US-A1- 2010 129 442

## Beschreibung

Die vorliegende Erfindung betrifft eine Arzneiform, ein Verfahren zu ihrer Herstellung und ihre Verwendungen.

### Stand der Technik

In der pharmazeutischen Technologie besteht ein fortwährender Bedarf an Arzneiformen, die eine gezielte Freisetzungscharakteristik von Wirkstoffen im menschlichen Gastrointestinaltrakt ermöglichen. Daher ist im Stand der Technik bereits eine Vielzahl verschiedener Arzneiformen bekannt. Beispielsweise gibt es monolithische Arzneiformen, die Wirkstoffe in einer polymeren Matrix enthalten. Nach Eintritt einer solchen Arzneiform in den Gastrointestinaltrakt des Menschen löst sich der Wirkstoff kontrolliert auf. Die Auflösungsrate kann insbesondere dadurch kontrolliert werden, dass ein Matrixpolymer ausgewählt wird, welches sich im Gastrointestinaltrakt nach und nach auflöst. Durch die Auflösung der Matrix wird der Wirkstoff freigegeben, löst sich in der vorhandenen Flüssigkeit und wird resorbiert. Ein anderer Ansatz sieht vor, dass ein Matrixpolymer verwendet wird, welches sich nicht auflöst, sondern den Wirkstoff über Poren freigibt. Wieder andere Konzepte bedienen sich osmotischer Systeme, welche die Wirkstofffreisetzung mithilfe einer Membran kontrollieren.

Die gerade erwähnten Arzneiformen setzen allerdings voraus, dass sich der Wirkstoff sowohl im sauren pH des Magens als auch im Darm, wo pH-Werte von typischerweise größer 6 vorherrschen, schnell löst. Löst sich der Wirkstoff langsamer als sich beispielsweise ein erodierendes Matrixpolymer auflöst, ist nicht mehr die Erosion des Matrixpolymers bestimmend für die Freigabe des Wirkstoffes, sondern die Lösungsgeschwindigkeit des Wirkstoffes. Gleiche Überlegungen treffen auf Arzneiformen zu, die poröse Matrices aufweisen oder auf osmotischen Systemen beruhen.

Besonders problematisch ist die kontrollierte Freisetzung von solchen Wirkstoffen, die schwache Basen sind. Schwache Basen sind im sauren Milieu des Magens protoniert und somit normalerweise positiv geladen. In dieser kationischen Form ist der schwach basische Wirkstoff gut wasserlöslich. Nachdem der Wirkstoff sich in diesem Milieu leicht löst, kann er zügig resorbiert werden. Tritt die herkömmliche Arzneiform mit kontrollierter Freisetzung allerdings in den Darm ein, befindet sie sich in einem weniger sauren Milieu. Folglich liegt der schwach basische Wirkstoff dort nicht mehr in der protonierten Form vor, sondern als ungeladenes Molekül. In dieser Form ist der Wirkstoff nur noch schlecht wasserlöslich. Hinzu kommt, dass die Flüssigkeitsmenge, die im Darm zur Auflösung des Wirkstoffes zur Verfügung steht, wesentlich geringer ist als im Magen. Das bedeutet, dass sich der Wirkstoff, sobald die Arzneiform im Darm angekommen ist, nur noch sehr schlecht löst, so dass die Auflösungsgeschwindigkeit des Wirkstoffes die Freigabe des Wirkstoffes aus der Arzneiform und damit auch die Resorptionsgeschwindigkeit bestimmt.

Im Stand der Technik sind einige pharmazeutische Darreichungsformen bekannt, die mehrere wirkstoffhaltige Einheiten enthalten:
EP 0 558 913 A1 beschreibt eine Darreichungsform, die zwei verschiedene Granulate A und B umfasst. Granulat A setzt den Wirkstoff Minocyclin in saurem Medium schnell frei und Granulat B enthält einen Überzug aus mindestens zwei Polymeren, von denen eines sich in Wasser schnell auflöst und eines sich erst ab einem pH-Wert von zwischen 4,5 und 6,5 auflöst. Der Überzug des Granulates B ist also nicht magensaftresistent, sondern setzt bereits im Magen Wirkstoff frei. Minocyclin ist gut in Wasser löslich, daher werden auch keine kleinen Partikelgrößen vorgeschlagen.

GB 2 414 668 A beschreibt Arzneiformen mit modifizierter Freisetzung, die zur Freisetzung von Tetracyclinen bestimmt sind. Die beschriebenen Arzneiformen umfassen zwei Tabletten, von denen eine den Wirkstoff schnell und eine verzögert freisetzt. Die Tabletten haben Durchmesser von mehr als 3 mm. Es wird nicht empfohlen, den Wirkstoff in Form kleiner Partikel einzusetzen.

WO 99/12524 A1 beschreibt Darreichungsformen mit modifizierter Freisetzung, die zwei Fraktionen von kleinen wirkstoffhaltigen Einheiten umfassen. Die erste Fraktion ist dazu bestimmt, einen Wirkstoff schnell freizusetzen. Eine zweite Fraktion ist mit einem Überzug versehen und setzt den Wirkstoff über einen längeren Zeitraum frei. Die Arzneiformen sind für nichtsteroidale Antiphlogistika (NSAIDs) bestimmt. NSAIDs sind typischerweise saure Substanzen, die sich im Magensaft schlechter lösen als im Darm. Mithin sind auch die Darreichungsformen auf dieses Lösungsverhalten abgestimmt.

WO 2004/112756 A1 beschreibt Kapseln, die Protonenpumpenhemmer in Untereinheiten aufweisen. Es ist vorgesehen, dass die Kapseln eine basische Substanz zum Schutz des Protonenpumpenhemmers umfassen. Es liegt auf der Hand, dass eine basische Substanz die Löslichkeit basischer Wirkstoffe behindert.

WO 2006/124898 A1 beschreibt Darreichungsformen, die Morphinsulfat in Pellets aufweisen. Allerdings wird hier nur eine wirkstoffhaltige Einheit beschrieben.

Die skizzierten Probleme wurden im Stand der Technik bereits mehrfach diskutiert. So beschreiben etwa WO 2006/0998865 A2 und WO 2006/099864 A2 Arzneiformen, die den schlecht löslichen Wirkstoff Cinnarizin umfasst. Es wird empfohlen, bestimmte Gewichtsverhältnis von Bindemittel zu Füllstoff und weiteren Inhaltsstoffen auf bestimmte Werte einzustellen, um die Freisetzung der in der Arzneiform enthaltenen Wirkstoffe synchron zu gestalten. Neuere Versuche haben allerdings gezeigt, dass eine synchrone Freisetzung allein für viele Arzneistoffe noch nicht ausreichend ist. Der Wirkstoff muss sich auch lösen, damit er resorbiert werden kann. Dieses Problem wurde damit nicht gelöst.

### Aufgabe

Es kann also festgestellt werden, dass herkömmliche Arzneiformen zur kontrollierten Freisetzung bei der Applikation schlecht löslicher Wirkstoffe an ihre Grenzen stoßen.

### Lösung

Die vorliegende Erfindung stellt Arzneiformen zur kontrollierten Freisetzung von Wirkstoffen zur Verfügung und löst damit das oben skizzierte Problem. Außerdem stellt die Erfindung ein Herstellungsverfahren für solche Arzneiformen und geeignete Verwendungen vor.

### Arzneiform

### Wirkstoffhaltige Einheiten

Die Arzneiform dieser Erfindung umfasst mindestens eine erste und mindestens eine zweite sowie optional mindestens eine dritte wirkstoffhaltige Einheit. Die wirkstoffhaltigen Einheiten sind vorzugsweise fest, insbesondere Pulver, Pellets oder Tabletten. Die wirkstoffhaltigen Einheiten, insbesondere die zweite, liegen dabei vorzugsweise als Pellets oder Tabletten, insbesondere Mikrotabletten, vor. Vorzugsweise enthält die Arzneiform eine Vielzahl zweiter und optional eine Vielzahl dritter wirkstoffhaltiger Einheiten. Auch die dritte wirkstoffhaltige Einheit liegt vorzugsweise als Pellet oder Tablette, insbesondere Mikrotablette, vor.

Die wirkstoffhaltigen Einheiten können auch in Form von Schichten in einer Schichttablette vorliegen. Erfindungsgemäße Arzneiformen können insbesondere Zweischichttabletten, Dreischichttabletten oder Mehrschichttabletten sein. Erfindungsgemäß sind auch Arzneiformen, in denen eine der wirkstoffhaltigen Einheiten, zum Beispiel die erste oder die zweite, in Form einer Schicht vorliegen und eine andere wirkstoffhaltige Einheit in Form von beispielweise Pellets oder Mikrotabletten in der Schicht vorliegen. Eine erfindungsgemäße Arzneiform kann beispielsweise auch so aufgebaut sein, dass eine wirkstoffhaltige Einheit als Schicht vorliegt, die zwischen zwei Schichten angeordnet ist. Die zwei Schichten können dann eine andere wirkstoffhaltige Einheit darstellen. Die zwischen den zwei Schichten eingebettete Schicht kann beispielsweise die erste oder die zweite wirkstoffhaltige Einheit darstellen.

Durch dieses Design wird es möglich, verschiedene Freisetzungscharakteristiken in einer Arzneiform zu verwirklichen, indem die unterschiedlichen Einheiten verschieden aufgebaut werden. Die verschiedenen Arten wirkstoffhaltiger Einheiten unterscheiden sich in ihrer Zusammensetzung. Vorzugsweise weisen die verschiedenen Arten wirkstoffhaltiger Einheiten voneinander abweichende Freisetzungscharakteristiken auf. Die Arzneiform umfasst vorzugsweise eine Vielzahl erster wirkstoffhaltiger Einheiten, eine Vielzahl zweiter wirkstoffhaltiger Einheiten und optional eine Vielzahl dritter wirkstoffhaltiger Einheiten.

Wenn in dieser Beschreibung der Begriff "wirkstoffhaltige Einheit" verwendet wird, so soll damit nicht zum Ausdruck gebracht werden, dass die erfindungsgemäße Arzneiform nur eine dieser Einheiten enthalten darf. Vielmehr enthalten die erfindungsgemäßen Arzneiformen in den meisten Fällen eine Vielzahl solcher Einheiten. Gleichwohl kann das erfindungsgemäße Ergebnis auch mit einer reduzierten Zahl wirkstoffhaltiger Einheiten erzielt werden.

Vorzugsweise weist wenigstens eine wirkstoffhaltige Einheit neben wenigstens einem Wirkstoff auch wenigstens einen Hilfsstoff auf, insbesondere weisen alle wirkstoffhaltigen Einheiten neben wenigstens einem Wirkstoff auch wenigstens einen Hilfsstoff auf.

Die erfindungsgemäße Arzneiform dient der kontrollierten Freisetzung von Wirkstoffen, die wenigstens bei vergleichsweise hohen pH-Werten schlechte Wasserlöslichkeit aufweisen. Dies bedeutet, dass sich diese Wirkstoffe im Milieu des Darms schlecht lösen. Um diese Wirkstoffe zur Resorption zu bringen, müssen sie allerdings gelöst werden.

Es hat sich als vorteilhaft erwiesen, wenigstens die zweite und die optionale dritte wirkstoffhaltige Einheit mit kleinem Durchmesser auszugestalten, um die Kontaktfläche der wirkstoffhaltigen Einheiten mit der vorhandenen Flüssigkeit im Gastrointestinaltrakt zu vergrößern. Um die Freisetzung des Wirkstoffes zu erleichtern, sollte der Durchmesser der wirkstoffhaltigen Einheiten, insbesondere der zweiten und der optionalen dritten Einheit, 7,5 mm vorzugsweise nicht überschreiten. Vorzugsweise beträgt der Durchmesser dieser wirkstoffhaltigen Einheiten nicht mehr als 5,5 mm und besonders bevorzugt nicht mehr als 3 mm, insbesondere beträgt der Durchmesser der wirkstoffhaltigen Einheiten kleiner 3 mm. Insbesondere sind wirkstoffhaltige Einheiten bevorzugt, die Durchmesser von nicht mehr als 2 mm und besonders bevorzugt nicht mehr als 1,8 mm aufweisen.

Sofern nichts anderes angegeben ist, ist mit "Durchmesser" derjenige Durchmesser gemeint, den die wirkstoffhaltigen Einheiten an ihrer dicksten Stelle aufweisen.

Werden die wirkstoffhaltigen Einheiten zu klein, wird die Herstellung und Handhabung erschwert. Deshalb beträgt der Durchmesser der wirkstoffhaltigen Einheiten, insbesondere der zweiten und optionalen dritten Einheit, vorzugsweise wenigstens 0,1 mm, ferner bevorzugt wenigstens 0,2 mm, insbesondere wenigstens 0,5 mm, und weiter bevorzugt wenigstens 1 mm. Durch diese kleinen Durchmesser der Einheiten wird erreicht, dass sich die enthaltenen Wirkstoffe oder der enthaltene Wirkstoff dort, wo wenig Flüssigkeit zur Verfügung steht, insbesondere im Darm, besser lösen. Es kann allerdings sinnvoll sein, alle wirkstoffhaltigen Einheiten mit dem oben beschriebenen kleinen Durchmesser auszugestalten, auch diese Ausführungsformen sind erfindungsgemäß.

Um eine möglichst große Oberfläche bereitzustellen und damit die Auflösungsgeschwindigkeiten der wirkstoffhaltigen Einheiten und damit des Wirkstoffes zu erhöhen, enthält die erfindungsgemäße Arzneiform vorzugsweise wenigstens 10 wirkstoffhaltige Einheiten. Vorzugsweise enthält die erfindungsgemäße Arzneiform sogar wenigstens 20, weiter bevorzugt wenigstens 30 und besonders bevorzugt wenigstens 40 wirkstoffhaltige Einheiten. Es sind auch Ausführungsformen erfindungsgemäß, die wenigstens 100 wirkstoffhaltige Einheiten aufweisen. Allerdings muss berücksichtigt werden, dass die erfindungsgemäße Arzneiform nicht zu groß sein darf, da ansonsten die Einnahme erschwert werden könnte. Daher enthält die erfindungsgemäße Arzneiform vorzugsweise höchstens 500 wirkstoffhaltige Einheiten. Vorzugsweise enthält die Arzneiform höchstens 400, weiter bevorzugt höchstens 200 und besonders bevorzugt höchstens 150 wirkstoffhaltige Einheiten.

Die wirkstoffhaltigen Einheiten, insbesondere die zweiten und optionalen dritten wirkstoffhaltigen Einheiten, haben vorzugsweise Oberflächen von jeweils wenigstens 5 mm² und vorzugsweise höchstens 2000 mm². Insbesondere haben diese wirkstoffhaltigen Einheiten jeweils Oberflächen von wenigstens 25 mm², weiter bevorzugt wenigstens 50 mm² und insbesondere höchstens 1500 mm² und weiter bevorzugt höchstens 1000 mm². Große Oberflächen erleichtern die Auflösung der Einheiten und damit die Auflösung und Resorption des Wirkstoffes oder der Wirkstoffe.

Grundsätzlich können die wirkstoffhaltigen Einheiten jede erdenkliche Form haben. Es hat sich allerdings als vorteilhaft erwiesen, die wirkstoffhaltigen Einheiten, insbesondere die zweite und die optionale dritte, in sphärischer (z.B. Pellets) oder zylindrischer Form (z.B. Tabletten) auszugestalten.

Insbesondere sind die zweite und die optionale dritte wirkstoffhaltige Einheit vorzugsweise feste Körper eines Volumens von jeweils wenigstens 1 mm³, bevorzugt wenigstens 3 mm³ und weiter bevorzugt wenigstens 5 mm³. Die Mindestgröße sollte eingehalten werden, um eine gute Verarbeitbarkeit zu ermöglichen. Um gutes Auflösungsverhalten sicherzustellen, sollte das Volumen der Einheiten einen Wert von bevorzugt 20 mm³, weiter bevorzugt 10 mm³ und mehr bevorzugt 8,5 mm³ nicht überschreiten.

Die erste wirkstoffhaltige Einheit hingegen kann in Form eines Pulvers oder einer Pulvermischung vorliegen. Dies ist deshalb möglich, weil die erste wirkstoffhaltige Einheit keine magensaftresistenten Eigenschaften aufweisen muss, da sie den ersten Wirkstoff möglichst sofort freisetzen soll.

In einer bevorzugten Ausführungsform handelt es sich bei den zweiten und optionalen dritten wirkstoffhaltigen Einheiten um Pellets, in einer anderen um Tabletten, insbesondere Mikrotabletten. In einer ebenfalls erfindungsgemäßen Ausführungsform können Pellets und Tabletten, insbesondere Mikrotabletten, in Kombination verwendet werden.

Die Zusammensetzung der Arzneiform aus wenigstens einer ersten, wenigstens einer zweiten und optional wenigstens einer dritten wirkstoffhaltigen Einheit ermöglicht die sequentielle Abgabe schlecht löslicher und/oder anderer Wirkstoffe, so dass eine mehrmals tägliche Gabe des oder der Wirkstoffe simuliert werden kann.

### Die zweite wirkstoffhaltige Einheit

Wie oben erwähnt umfasst die Arzneiform wenigstens eine erste und wenigstens eine zweite wirkstoffhaltige Einheit, insbesondere eine Vielzahl zweiter wirkstoffhaltiger Einheiten. Der damit verbundene Vorteil liegt in der Möglichkeit der flexiblen Anpassung der verschiedenen wirkstoffhaltigen Einheiten an das Löslichkeitsverhalten des Wirkstoffes oder der Wirkstoffe.

In einer bevorzugten Ausführungsform ist die erste wirkstoffhaltige Einheit so ausgestaltet, dass sie sich bei Kontakt mit dem Magensaft schnell auflöst und den ersten Wirkstoff, und optionale weitere Wirkstoffe, freigibt. Ist der erste Wirkstoff, wie erfindungsgemäß bevorzugt, eine schwache Base, wird er im sauren Milieu des Magens überwiegend protoniert, so dass er sich auflöst und resorbiert werden kann. Dadurch wird sehr schnell nach der Einnahme eine entsprechende Menge an Wirkstoff resorbiert, während derjenige Anteil des Wirkstoffes, der sich in der zweiten und optionalen dritten Einheit befindet, davon unbeeinflusst bleibt.

Es ist bevorzugt, dass die zweite wirkstoffhaltige Einheit so ausgestaltet ist, dass der erste Wirkstoff aus dieser Einheit im Magen noch nicht freigesetzt wird. Dies wird vorzugsweise dadurch erzielt, dass die zweite Einheit ein Polymer umfasst, welches sich im Milieu des Magens nicht auflöst. Der erste Wirkstoff bleibt somit in der zweiten wirkstoffhaltigen Einheit vor der Flüssigkeit im Magen geschützt, löst sich nicht auf und wird nicht resorbiert. Dieses Polymer bildet eine Matrix aus, in welcher der erste Wirkstoff dispergiert ist (im Folgenden: "Wirkstoffmatrix"). Das Polymer der Matrix wird nachfolgend "Matrixpolymer" genannt.

Das Matrixpolymer kann magensaftlöslich oder magensaftunlöslich sein. Ist es wasserunlöslich, muss die zweite wirkstoffhaltige Einheit keinen magensaftresistenten Überzug aufweisen, weil das Matrixpolymer allein die Freisetzung verhindern kann. Ist das Matrixpolymer hingegen magensaftlöslich, ist es bevorzugt, die zweite wirkstoffhaltige Einheit mit einem magensaftresistenten Überzug zu versehen.

Folglich kann die zweite wirkstoffhaltige Einheit mit einem Polymer, insbesondere einem magensaftunlöslichen Polymer, überzogen sein. Entsprechende Polymere sind dem Fachmann bekannt und werden im Folgenden "Überzugspolymere" genannt.

### Überzug

Bevorzugt wird die zweite wirkstoffhaltige Einheit mit einem Überzug eines Überzugspolymers versehen. Dieser Überzug umfasst vorzugsweise ein Überzugspolymer, welches Säuregruppen aufweist, die im deprotonierten Zustand geladen sind. Überzugspolymere, die solche Säuregruppen aufweisen, lösen sich im basischen Milieu des Darms besser als andere Polymere. Ferner lösen sie sich im sauren Milieu des Magens nicht auf, sie sind magensaftunlöslich. Vorzugsweise weist das Überzugspolymer pro Monomer wenigstens 0,3 Säurefunktionen auf. Die Säurefunktion kann eine Carbonsäuregruppe sein. In besonders bevorzugten Ausführungsformen ist das Überzugspolymer ein Polymer, das Methacrylsäuremonomere umfasst, wie insbesondere Eudragit® L-100-55.

Weitere bevorzugte Überzugspolymere sind Cellulosederivate, insbesondere solche, die mit organischen Säuren verestert sind. Die organischen Säuren können aromatisch und/oder aliphatisch sein, wobei in einer bevorzugten Ausführungsform ein Cellulosederivat eingesetzt wird, dass sowohl aromatisch als auch aliphatisch verestert ist. Die organischen Säuren sollten vorzugsweise nicht mehr als 10 Kohlenstoffatome aufweisen, vorzugsweise sollten sie aber wenigstens 2 Kohlenstoffatome umfassen. In einer erfindungsgemäßen Ausführungsform ist das Überzugspolymer Celluloseacetatphthalat.

Ferner können als Cellulosederivate auch Celluloseether verwendet werden. In diesem Fall ist es bevorzugt, Cellulose, die mit kurzkettigen Alkylresten verethert ist, zu verwenden. "Kurzkettig" bedeutet, dass die Alkylreste vorzugsweise bis zu vier Kohlenstoffatome aufweisen. Die Alkylreste sind ferner vorzugsweise unverzweigt. Ein besonders bevorzugter Celluloseether ist Ethylcellulose.

Ferner kann Schelllack als Überzugspolymer verwendet werden.

Dem Überzugspolymer wird vorzugsweise ein Weichmacher zugesetzt, bei dem es sich um Glycerintriacetat handeln kann.

Insbesondere enthält der Überzug der zweiten wirkstoffhaltigen Einheit vorzugsweise ein Überzugspolymer, welches sich im oberen Darmabschnitt bei einem pH-Wert von wenigstens 6 auflöst. Dem Fachmann sind solche Überzugspolymere bekannt. In einer Ausführungsform wird ein Überzugspolymer verwendet, welches ein Copolymer aus Methacrylsäure- und Alkylmethacrylatmonomeren ist. Dabei ist das Stoffmengenverhältnis von Säuremonomeren zu Estermonomeren, welche die Ausgangsstoffe dieses Überzugspolymers darstellen, vorzugsweise wenigstens 45:55 und besonders bevorzugt höchstens 70:30.

Durch diesen Überzug wird gewährleistet, dass die zweite wirkstoffhaltige Einheit sich erst bei einem pH von wenigstens 6 auflöst. Ein solcher pH-Wert herrscht typischerweise im oberen Darmabschnitt (Jejunum) vor.

Das Design der erfindungsgemäßen Arzneiform erlaubt es also, eine Freisetzungscharakteristik einzustellen, die mehr als eine Freisetzungsphase umfasst. Nach der ersten Freisetzungsphase im Magen - vermittelt durch die erste wirkstoffhaltige Einheit -, wird im oberen Darmabschnitt aufgrund des Zerfalls der zweiten wirkstoffhaltigen Einheit eine zweite Freisetzungsphase stattfinden, wodurch eine deutlich verlängerte Freisetzung ermöglicht wird.

Erfindungsgemäß besteht der Überzug der zweiten wirkstoffhaltigen Einheit vorzugsweise zu wenigstens 90 Gew.-% aus dem Überzugspolymer und dem Weichmacher. Bevorzugt besteht der Überzug zu wenigstens 95 Gew.-%, weiter bevorzugt zu wenigstens 98 Gew.-% aus diesen beiden Komponenten.

### Wirkstoffmatrix

Da im menschlichen Darm nur geringe Flüssigkeitsmengen zur Verfügung stehen, empfiehlt es sich, die Auflösung des ersten Wirkstoffes im Darm zu unterstützen. Dies kann durch Bereitstellung einer Vielzahl kleiner wirkstoffhaltiger Einheiten wie oben beschrieben erzielt werden. Alternativ oder zusätzlich kann der erste Wirkstoff in der zweiten wirkstoffhaltigen Einheit fein dispergiert vorliegen. "Fein dispergiert" bedeutet, dass der Wirkstoff in Form kleiner Partikel in einer Wirkstoffmatrix verteilt vorliegt.

Wenn diese wirkstoffhaltige Einheit einen Überzug aufweist, kann die Wirkstoffmatrix, wie oben beschrieben, aus demselben Material wie der Überzug oder aus einem anderen Material bestehen.

Ferner umfasst die zweite wirkstoffhaltige Einheit eine Dispersion des ersten Wirkstoffes in einer Wirkstoffmatrix. Die Wirkstoffmatrix umfasst dann den dispergierten Wirkstoff und ein Dispersionsmittel.

Das Dispersionsmittel kann ein Matrixpolymer umfassen oder daraus bestehen. Das Matrixpolymer ist vorzugsweise ein wasserlösliches Polymer. Bevorzugte wasserlösliche Matrixpolymere sind Polyvinylpyrrolidon, wasserlösliche Cellulosen, Polyethylenglykole und Poloxamere. Besonders bevorzugt sind Hydroxypropylmethylcellulose und Polyethylenglykole, insbesondere mit mittleren Molekülmassen von 6000 bis 20000.

Alternativ kann das Matrixpolymer dasselbe Polymer sein, welches auch für den Überzug dieser wirkstoffhaltigen Einheit verwendet wird. Wenn PVP als Matrixpolymer verwendet wird, sollte es eine mittlere Molekülmasse in einem Bereich von 10000 bis 100000 aufweisen, besonders bevorzugt sind 25000 bis 60000.

Es hat sich gezeigt, dass das Auflösungsverhalten des ersten Wirkstoffes wesentlich verbessert wird, wenn das Masseverhältnis von erstem Wirkstoff zu Matrixpolymer in der zweiten wirkstoffhaltigen Einheit, und vorzugsweise auch in der optionalen dritten Einheit, einen Wert von höchstens 1:1 annimmt, der Wirkstoff ist also höchstens in einer Menge vorhanden, die der Masse des Matrixpolymers entspricht. Besonders bevorzugt beträgt dieses Verhältnis höchstens 1:2 und weiter bevorzugt höchstens 1:2,3. Ist der Anteil des Matrixpolymers in diesem Verhältnis geringer, kann das Matrixpolymer die Auflösung des ersten Wirkstoffes nicht mehr ausreichend fördern. Ist die Menge des Matrixpolymers im Vergleich zum Wirkstoff größer, so wird die Auflösung auch schlecht löslicher Wirkstoffe stark verbessert.

Grundsätzlich kann die Menge an Matrixpolymer im Rahmen einer dem Fachmann sinnvoll erscheinenden Spanne beliebig hoch gewählt werden. Damit die Masse der wirkstoffhaltigen Einheit und damit der ganzen Arzneiform allerdings nicht zu hoch wird, sollte das Verhältnis von Wirkstoff zu Matrixpolymer einen Wert von 1:15 nicht unterschreiten. In bevorzugten Ausführungsformen beträgt die Untergrenze dieses Verhältnisses wenigstens 1:10.

Ferner kann die Wirkstoffmatrix ein Komplexierungsmittel umfassen. Bevorzugte Komplexierungsmittel sind Cyclodextrine. Auch Fettsäureester, insbesondere Sucrose-Fettsäureester können in der zweiten wirkstoffhaltigen Einheit enthalten sein. Die aufgezählten Stoffe können in der Wirkstoffmatrix enthalten sein und haben einen positiven Einfluss auf die Löslichkeit des ersten Wirkstoffes.

In bevorzugten Ausführungsformen besteht die Wirkstoffmatrix zu wenigstens 50 Gew.-%, weiter bevorzugt wenigstens 70 Gew.-% und besonders bevorzugt wenigstens 90 Gew.-% aus einem oder mehreren der oben erwähnten Stoffe (Komplexierungsmittel, Fettsäureester, Matrixpolymer), insbesondere aus einem oder mehreren der erwähnten Polymere.

Zusammengefasst umfasst die zweite wirkstoffhaltige Einheit also eine Wirkstoffmatrix, in welcher der erste Wirkstoff dispergiert ist. Ferner umfasst die wirkstoffhaltige Einheit vorzugsweise einen Überzug, welcher ein Überzugspolymer umfasst. Außerdem kann die zweite wirkstoffhaltige Einheit einen zweiten Wirkstoff umfassen.

Die erfindungsgemäße Arzneiform umfasst wenigstens eine wirkstoffhaltige Einheit dieser Art, vorzugsweise umfasst sie jedoch eine Vielzahl dieser Einheiten, nämlich insbesondere wenigstens 10, weiter bevorzugt wenigstens 30, mehr bevorzugt wenigstens 100.

Die zweite wirkstoffhaltige Einheit liegt vorzugsweise in Form eines Pellets, einer Schicht in einer Schichttablette oder einer Tablette vor. Dies trifft vorzugsweise auch auf die optionale dritte wirkstoffhaltige Einheit zu. In besonders bevorzugten Ausführungsformen unterscheidet sich die optionale dritte wirkstoffhaltige Einheit lediglich im Hinblick auf das Matrixpolymer und/oder das Überzugspolymer von der zweiten wirkstoffhaltigen Einheit.

### Die dritte wirkstoffhaltige Einheit

In besonders bevorzugten Ausführungsformen weist die erfindungsgemäße Arzneiform neben der ersten und zweiten wirkstoffhaltigen Einheit zusätzlich wenigstens eine dritte wirkstoffhaltige Einheit auf.

Die dritte wirkstoffhaltige Einheit ist vorzugsweise im Wesentlichen so ausgestaltet wie die zweite wirkstoffhaltige Einheit mit der Maßgabe, dass diese Einheit dazu bestimmt ist, den ersten Wirkstoff im unteren Darmabschnitt (Ileum, Colon) freizusetzen.

Im unteren Darmabschnitt herrschen höhere pH-Werte als im oberen Darmabschnitt. Daher wird die dritte wirkstoffhaltige Einheit vorzugsweise mit einem Überzug versehen, der sich erst bei pH-Werten von wenigstens 7 auflöst. Dem Fachmann sind solche Überzüge bekannt. Insbesondere kann es sich bei so einem Überzug um einen polymeren Überzug handeln. Vorzugsweise wird dieser Überzug durch Mischung von Eudragit® L100 und Eudragit® S100 hergestellt. Das Mischungsverhältnis beträgt dabei vorzugsweise 1 zu 10 bis 5 zu 10 bezogen auf die Masse.

Eudragit® L100 entspricht Poly(methacrylsäure-co-methyl methacrylat) 1:1 (CAS 25086-15-1). Eudragit® S100 entspricht Poly(methacrylsäure-co-methylmethacrylat) 1:2 (CAS 25086-15-1). Eudragit® L100-55 entspricht Poly(methacrylsäure-co-ethylacrylat) 1:1 (CAS 25212-88-8).

Erfindungsgemäß besteht der Überzug der dritten wirkstoffhaltigen Einheit vorzugsweise zu wenigstens 90 Gew.-% aus dem Überzugspolymer und dem Weichmacher. Bevorzugt besteht der Überzug zu wenigstens 95 Gew.-%, weiter bevorzugt zu wenigstens 98 Gew.-% aus diesen beiden Komponenten.

Insbesondere weist die dritte wirkstoffhaltige Einheit einen Überzug auf, der ein Überzugspolymer enthält, welches sich im unteren Darmabschnitt bei einem pH-Wert von wenigstens 7 auflöst. Dem Fachmann sind solche Überzugspolymere bekannt. Vorzugsweise wird ein Überzugspolymer verwendet, welches ein Copolymer aus Methacrylsäure- und Alkylmethacrylatmonomeren ist. Dabei ist das Stoffmengenverhältnis von Säuremonomeren zu Estermonomeren vorzugsweise wenigstens 25:75 und höchstens 40:60. Ferner kann dieses Polymer auch als Matrixpolymer verwendet werden, sodass die dritte wirkstoffhaltige Einheit eine Matrix aus diesem Polymer aufweist. Der Wirkstoff ist dann vorzugsweise in der Matrix dispergiert. Bevorzugte Matrixpolymere in der dritten wirkstoffhaltigen Einheit sind wasserlösliche Polymere. Bevorzugte wasserlösliche Matrixpolymere sind Polyvinylpyrrolidon, wasserlösliche Cellulosen, Polyethylenglykole und Poloxamere. Besonders bevorzugt sind Hydroxypropylmethylcellulose und Polyethylenglykole, insbesondere mit mittleren Molekülmassen von 6000 bis 20000.

Alternativ kann das Matrixpolymer dasselbe Polymer sein, welches auch für den Überzug dieser wirkstoffhaltigen Einheit verwendet wird. Wenn PVP als Matrixpolymer verwendet wird, sollte es eine mittlere Molekülmasse in einem Bereich von 10000 bis 100000 aufweisen, besonders bevorzugt sind 25000 bis 60000.

Es hat sich gezeigt, dass das Auflösungsverhalten des ersten Wirkstoffes wesentlich verbessert werden kann, wenn das Masseverhältnis von erstem Wirkstoff zu Matrixpolymer in der dritten wirkstoffhaltigen Einheit einen Wert von höchstens 1:1 annimmt, der Wirkstoff ist also höchstens in einer Menge vorhanden, die der Masse des Matrixpolymers entspricht. Besonders bevorzugt beträgt dieses Verhältnis höchstens 1:2 und weiter bevorzugt höchstens 1:2,3. Ist der Anteil des Matrixpolymers in diesem Verhältnis geringer, kann das Matrixpolymer die Auflösung des ersten Wirkstoffes oft nicht mehr ausreichend fördern. Ist die Menge des Matrixpolymers im Vergleich zum Wirkstoff größer, so wird die Auflösung auch schlecht löslicher Wirkstoffe stark verbessert. Grundsätzlich kann die Menge an Matrixpolymer im Rahmen einer dem Fachmann sinnvoll erscheinenden Spanne beliebig hoch gewählt werden. Damit die Masse der wirkstoffhaltigen Einheit und damit der ganzen Arzneiform allerdings nicht zu hoch wird, sollte das Verhältnis von Wirkstoff zu Matrixpolymer einen Wert von 1:15 nicht unterschreiten. In bevorzugten Ausführungsformen beträgt die Untergrenze dieses Verhältnisses wenigstens 1:10.

Ferner kann die dritte wirkstoffhaltige Einheit ein Komplexierungsmittel umfassen. Bevorzugtes Komplexierungsmittel sind Cyclodextrine. Auch Fettsäureester, insbesondere Sucrose-Fettsäureester können in der dritten wirkstoffhaltigen Einheit enthalten sein. Die aufgezählten Stoffe können in der Wirkstoffmatrix enthalten sein und haben einen positiven Einfluss auf die Löslichkeit des ersten Wirkstoffes. In bevorzugten Ausführungsformen besteht die Wirkstoffmatrix zu wenigstens 50 Gew.-%, weiter bevorzugt wenigstens 70 Gew.-% und besonders bevorzugt wenigstens 90 Gew.-% aus einem oder mehreren der oben erwähnten Stoffe (Komplexierungsmittel, Fettsäureester, Polymer), insbesondere aus einem oder mehreren der erwähnten Polymere.

Da sich zweite und dritte wirkstoffhaltige Einheit in einer bevorzugten Ausführungsform nur durch ihr Überzugspolymer voneinander unterscheiden, gilt das für die zweite wirkstoffhaltige Einheit oben Erwähnte auch für die dritte wirkstoffhaltige Einheit.

Zusammengefasst umfasst die dritte wirkstoffhaltige Einheit also bevorzugt eine Wirkstoffmatrix, in welcher der erste Wirkstoff dispergiert ist. Ferner umfasst die wirkstoffhaltige Einheit vorzugsweise einen Überzug, welcher ein Überzugspolymer umfasst. Außerdem kann die zweite wirkstoffhaltige Einheit einen zweiten Wirkstoff umfassen.

Die erfindungsgemäße Arzneiform umfasst wenigstens eine wirkstoffhaltige Einheit dieser Art, vorzugsweise umfasst sie jedoch eine Vielzahl dieser Einheiten, nämlich insbesondere wenigstens 10, weiter bevorzugt wenigstens 30, mehr bevorzugt wenigstens 100.

Die dritte wirkstoffhaltige Einheit liegt vorzugsweise in Form eines Pellets, einer Schicht in einer Schichttablette oder einer Tablette vor.

### Der erste Wirkstoff

Die wirkstoffhaltigen Einheiten enthalten wenigstens einen ersten Wirkstoff. Der erste Wirkstoff ist bevorzugt schwach basisch. "Schwach basisch" bedeutet, dass der erste Wirkstoff einen pK_{B}-Wert von nicht weniger als 2,0 aufweist. Daher ist der erste Wirkstoff in der erfindungsgemäßen Arzneiform ein Wirkstoff, der wenigstens eine Aminogruppe umfasst. Der erste Wirkstoff weist bei einem pH-Wert von 1,1 eine Löslichkeit in Wasser von weniger als 10 mg/ml auf.

"Schlecht löslich" und "unlöslich" soll in dieser Beschreibung im Zweifelsfall und vorzugsweise bedeuten, dass die so beschriebene Substanz in dem entsprechenden Medium eine Löslichkeit von weniger als 0,5 Mol/l, insbesondere weniger als 0,1 Mol/l aufweist. "Magensaftunlöslich" soll im Zweifelsfall und vorzugsweise bedeuten, dass die so bezeichnete Substanz in einer wässrigen Lösung mit einem pH-Wert von 1,1 eine Löslichkeit von weniger als 0,5 Mol/l, insbesondere weniger als 0,1 Mol/l aufweist.

"Löslich" oder "gut löslich" beschreibt vorzugsweise Substanzen, die bessere Löslichkeitseigenschaften aufweisen als oben unter "schlecht löslich" und "unlöslich" beschrieben.

Wird in dieser Beschreibung der Begriff "Vielzahl" verwendet, so ist damit im Zweifelsfall eine Anzahl von mehr als 10 gemeint.

Der erste Wirkstoff ist ausgewählt aus Wirkstoffen mit schlechter Löslichkeit in wässrigen Medien, auch im sauren und/oder alkalischen Milieu, es handelt sich dabei um Wirkstoffe aus der Gruppe der Antihistaminika und/oder Calciumantagonisten. In einer besonders bevorzugten Ausführungsform ist der erste Wirkstoff Cinnarizin oder ein Salz des Cinnarizins.

In bevorzugten Ausführungsformen umfasst die erfindungsgemäße Arzneiform keine Antibiotika in pharmazeutisch wirksamer Menge, insbesondere keinen Vertreter der Wirkstoffklasse der Tetracycline. Antibiotika beeinträchtigen die Darmflora, in dem sie wichtige Bakterien im Darm abtöten. In der Arzneiform dieser Erfindung würde dieser Effekt zu starken Nebenwirkungen führen, weil die Arzneiform sich teilweise erst sehr spät auflöst.

In bevorzugten Ausführungsformen umfasst die erfindungsgemäße Arzneiform keine Protonenpumpenhemmer. Protonenpumpenhemmer erhöhen den pH-Wert im Magen und stören somit die Auflösung vieler Arzneistoffe aus der ersten wirkstoffhaltigen Einheit, insbesondere der schwach basischen Wirkstoffe.

Der Wirkstoff ist bevorzugt in Form kleiner Partikel in der Wirkstoffmatrix dispergiert. Das bedeutet, dass die mittlere Partikelgröße des ersten Wirkstoffes in der zweiten wirkstoffhaltigen Einheit, und insbesondere auch in der optionalen dritten wirkstoffhaltigen Einheit, vorzugsweise 1000 nm nicht übersteigt. Weiter bevorzugt beträgt die mittlere Partikelgröße des ersten Wirkstoffes in der zweiten wirkstoffhaltigen Einheit, und insbesondere auch in der dritten wirkstoffhaltigen Einheit, nicht mehr als 300 nm.

Die Partikelgröße des Wirkstoffes wird wie folgt bestimmt: die Matrix wird in einem Medium aufgelöst, in dem die Wirkstoffpartikel unlöslich sind. Aus dieser Suspension wird mit mittels Laserdiffraktion die Partikelgrößenverteilung bestimmt.

Sofern in dieser Beschreibung "Partikelgrößen" erwähnt werden und nichts anderes angegeben ist, ist im Zweifel stets der Ferret'sche Durchmesser gemeint, wie er beispielsweise im Wege mikroskopischer Methoden bestimmt werden kann.

### Aufbau der Arzneiform

Die erfindungsgemäße Arzneiform ist vorzugsweise so ausgestaltet, dass sie die wirkstoffhaltigen Einheiten nach Einnahme der Arzneiform durch den Patienten freigibt. Dies kann grundsätzlich auf verschiedene Arten verwirklicht werden. Die Arzneiform kann beispielsweise eine Trägermatrix aufweisen, in welche die wirkstoffhaltigen Einheiten eingebettet sind. Die Trägermatrix ist dann vorzugsweise so ausgewählt, dass sie sich bei Kontakt mit dem Magensaft sehr schnell auflöst. Dem Fachmann sind entsprechende Matrixmaterialien bekannt, insbesondere kann es sich dabei um wasserlösliche Polymere handeln. Beispielsweise können dies Polymere sein, die weiter oben als wasserlöslich beschrieben wurden.

In einer erfindungsgemäß bevorzugten Ausführungsform weist die Arzneiform eine Hülle auf, welche die wirkstoffhaltigen Einheiten umschließt. Die Hülle ist vorzugsweise so ausgestaltet, dass sie sich bei Kontakt mit dem Magensaft sehr schnell auflöst. Insbesondere kann es sich bei der Hülle um die Hülle einer in der pharmazeutischen Technologie üblicherweise verwendeten Hartkapsel handeln. Bevorzugte Materialien, aus denen die Hülle gefertigt werden kann, sind wasserlösliche Polymere, insbesondere Gelatine.

Es sind auch Arzneiformen erfindungsgemäß, die keine Hülle aufweisen. Insbesondere können die wirkstoffhaltigen Einheiten in einem Beutel enthalten sein, der vom Patienten geöffnet wird und dessen Inhalt eingenommen werden kann. Die Arzneiform kann auch in Form von Sticks vorliegen, welche die wirkstoffhaltigen Einheiten enthalten.

Ferner können die wirkstoffhaltigen Einheiten auch zu einem Körper komprimiert werden, so dass die Arzneiform in Form eines MUPS (Multiple Unit Pellet System) vorliegt.

### Der zweite Wirkstoff

Neben dem ersten Wirkstoff enthält die erfindungsgemäße Arzneiform vorzugsweise einen zweiten Wirkstoff. Der zweite Wirkstoff kann gute Löslichkeit in Wasser aufweisen. Es ist erfindungsgemäß bevorzugt, dass der zweite Wirkstoff eine permanente Ladung aufweist, wodurch ihm die gute Wasserlöslichkeit verliehen wird. Ein bevorzugtes Beispiel eines solchen Wirkstoffes ist Dimenhydrinat. Es können aber auch andere Wirkstoffe mit vergleichbarer therapeutischer Relevanz verwendet werden.

Beispiele für Wirkstoffe und Wirkstoffklassen, welche vorteilhaft als zweiter Wirkstoff im Rahmen der vorliegenden Erfindung eingesetzt werden können, sind:
Arzneimittel zur Behandlung von Schmerzen und zur Schmerztherapie mit peripher wirkenden Analgetika, zentral wirkenden Analgetika und adjuvante Nicht-Analgetika. Es handelt sich hier vorzugsweise um folgende Analgetika und adjuvante Stoffe:
Acetylsalicylsäure, Ibuprofen, Diclofenac, Indomethacin, Naproxen, Piroxicam, Paracetamol, Metamizol, Celecoxib, Parecoxib, Tramadol, Pethidin, Codein, Dihydrocodein, Piritramid, Tilidin, Morphin, Hydromorphon, Oxycodon, Levomethadon, Fentanyl, Sufentanil, Buprenorphin, Pentazocin, Naloxon, Flupirtin, Carbamazepin, Metoprolol, Metoclopramid, Amitryptilin, Doxepin, Clomipramin, Mianserin, Maprotilin, Triptane wie z.B. Naratriptan, Rizatriptan, Sumatriptan, Zolmitriptan, Kalziumantagonisten wie: Flunarizin, Topiramat, Valproinsäure, Phenytoin, Baclofen, sonstige Mittel wie: Botulinum Toxin, Ergotamine, Lisurid, Methysergid, Pizotifen, Oxcarbazepin, Gabapentin und Lamotrigin, Dexamethaon, Methylprednisolon, Prednisolon, Triamcinolon, Diazepam, Tetrazepam, Tizanidin, Butylscopolamin und/oder Kombination von Tilidin und Naloxon.

Arzneistoffe zur Behandlung des Nervensystems, allein oder in Kombination, z.B. Anfallsleiden (insbesondere Clonazepam, Diazepam, Lorazepam, Midazolam, Clobazam, Phenytoin, Clomethiazol, Valproinsäure, Phenobarbital, Gabapentin, Lamotrigin, Oxcarbazepin, Pregabalin, Topiramat, Ethosuximid, Levetrazetam, Mesuximid, Primidon, Nitrazepam und/oder Vigabitrin), Parkinson-Syndrom (insbesondere Levodopa, mit Benserazid/Carbidopa, Bromocriptin, Cabergolin, Dihydroergocriptin, Lisurid, Pergolidmesilat, Pramipexol, Ropinirol, Apomorphin, Biperiden, Metixenhydrochlorid, Trihexphenidyl, Entacapon, Amantadin, Bupidin, Selegilin und/oder Apomorphin), Schlaganfall (insbesondere Acetylsalicylsäure, Clopidogrel, Dipyridamol, Ticlopidin, Heparin, Phenprocoumon, Warfarin, Protamin, Phytomenadion, Nimodipin, Paracetamol, Tramadol und/oder Buprenorphin), Hirndruck (insbesondere Furosemid und/oder Mannitol), Tremor (insbesondere Propranolol, Clozapin, Alprazolam und/oder Primidon).

Arzneistoffe zur Behandlung von physchiatrischen Erkrankungen wie Angststörungen (insbesondere Alprazolam, Diazepam, Fluoxetin, Paroxetin, Chlorprothixen, Levomepromazin, Thi-oridazin, Flupentixol und/oder Fluspirilen), Depressionen (insbesondere Imipramin, Amitripytlin, Desipramin, Maprotilin, Minaserin, Citalopram, Fluoxetin, Paroxetin, Trazodon, Moclobemid und/oder Miratazepam), Psychosen und Schizophrenien (insbesondere Sulpirid, Promazin, Melperon, Thioridazin, Chlorprothixen, Perazin, Pimozid, Fluphenazin, Olanzapin und/oder Risperidon), Schlafstörungen (insbesondere Triazolam, Brotizolam, Oxazepam, Flurazepam, Nitrazepam, Temazepam, Zolpidemtratrat, Zopiclon, Promethazin, Chlorprothixen, Pipamperon, Thioridazin und/oder Chloralhydrat) Unruhezustände und Verwirrtheit (insbesondere Alprazolam, Oxazepam, Doxepin, Clomipramin, Imipramin, Thioridazin und/oder Perazin), Demenz vom Alzheimer-Typ (insbesondere Donepezil, Rivastigmin, Tacrin, Memantin, Nimodipin und/oder Seleginin).

Arzneistoffe zur Behandlung von Herz-Kreislauf-Krankheiten, wie koronare Herzkrankheit/Angina Pectoris (insbesondere Acetylsalicylsäure, Clopidrogel, Ticlopidin, Isosorbiddinitrat, Isosorbidmononitrat, Nitroglycerin, Molsidomin, Trapidil, Metoprolol, Bisoprolol, Atenolol, Acebutolol, Carvedilol, Nitrendipin, Nifedipin, Diltiazem, Verapamil, Benazepril, Lisinopril, Ramipril, Fosinopril und/oder Enalapril), Herzinfarkt und Herzinsuffizienz (insbesondere Isosorbidmono- und dinitrat, Clopidogrel, Ticlopidin, Captoprol, Ramipril, Lisinopril, Candesartan, Eproartan, Irbesatan, Losartan, Chlortalidon, Xipamid, Hydrochlorothiazid, Furosemid, Piretanid, Triameteren, Digitalisglycoside, Carvedilol, Metoprolol und/oder Prazosin), Herzrhythmusstörungen (insbesondere Ajmalin, Chinidin, Disopyramid, Flecainid, Propafenon, Propranolol, Carvedilol, Amiodaron, Verapamil und/oder Diltiazem), Hypertonie (insbesondere Metoprolol, Atenolol, Urapidil, Clonidin, Dihydralazin, Chlortalidon, Hydrochlorothiazid, Furosemid, Felodipin, Israpidin, Lacidipin, Diltiazem, Captopril, Enalapril, Fosinopril, Lisinopril, Ramnipril, Verapamil, Candesartan, Eprosartan, Irbesatan, Losartan, Doxazosin, Bunazosin, Prazosin, Terazosin, Moxonidin, Dihydralazin und/oder Minoxidil).

Arzneistoffe, allein oder in Kombination, zur Behandlung der Atemwege und der Lunge (insbesondere Theophyllin, Methylprednisolon, Flucorton, Dexamethason, Montekulast, Roxithromycin, Erythromycin, Azithromycin, Ciprofloxacin, Clarithromycin, Levofloxacin, Ofloxacin, Doxycyclin, Ampicillin und Sulbactam, Amoxicillin, Cefuroxim, Clindamycin, Cefotiam, Cefuroxim, Ceftazidim, Ceftriaxon, Piperacillin und/oder Moxifloxacin).

Arzneistoffe zur Behandlung des Magen-Darm-Traktes und der Bauchspeicheldrüse (insbesondere Fluconazol, Mesalazin, Sulfasalazin, Budenosid, Azathioprin, Prednison, Metronidazol, Infliximab, Loperamid, Cotrimoxazol, Ciprofloxacin, Metronidazol, Vancomycin, Esomeprazol, Lansoparzol, Pantoprazol, Rabeprazol, Cimetidin, Famotidin, Ranitidin, Nizatidin, Sucralfat, Misoprostol, Metoclopramid, Pirenzepin, Bisacodyl, Domperidon, Sulpirid, Alizaprid, Dimenhydrinat, Cinnarizin, Flunarizin, Levomeprazin, Ondansetron, Betahistin und/oder Aprepitant).

Arzneistoffe zur Infektabwehr mit Antibiotika oder antiviral wirksamen Substanzen (insbesondere Acyclovir, Amantadin, Azithromycin, Bacampicillin, Cefaclor, Cefazolin, Cefixim, Cefprozil, Ceftriaxon, Chloroquin, Ciprofloxacin, Clotrimazol, Dicloxacillin, Doxycycllin, Econazol, Erythromycin, Ethambutol, Fosfomycin, Flucloxacillin, Fluconazol, Fusidinsäure, Gramicidin, Idoxuridin, Indinavir, Interferon, Itraconazol, Isoniazid, Josamycin, Ketoconazol, Lamivudin, Lomefloxacin, Mafenid, Mebendazol, Mesalazin, Mezlozillin, Mupirocin, Miconazol, Naftifin, Nalidixinsäure, Norfloxacin, Ofloxacin, Oxacillin, Oxytetracyclin, Piperacillin, Praziquantel, Primaquim, Proguanil, Ribavirin, Rifabutin, Rimantadin, Roxothromycin, Saquinavir, Spectinomycin, Spriramycin, Stavudin, Sulbactam, Teiucoplanin, Terbinafin, Tetracyclin, Tetroxoprim, Ticarcillin, Tinidazol, Tromantadin, Tolnaftat, Vancomycin, Zidovudin und/oder Zalcitabin).

Arzneistoffe zur Behandlung der erektilen Dysfunktion (insbesondere Sildenafil, Tadalafil, Vardenafil, Theobromin, Koffein und/oder Theophyllin)..

Der zweite Wirkstoff kann, insbesondere wenn es sich um einen Wirkstoff mit langer Halbwertzeit handelt, beispielsweise nur in der ersten wirkstoffhaltigen Einheit enthalten sein. Bei solchen Wirkstoffen ist, aufgrund der langen Wirkdauer nach der Einnahme, eine kontrollierte mehrmalige Freisetzung nicht erforderlich.

In anderen Ausführungsformen, insbesondere wenn der zweite Wirkstoff eine kürzere Halbwertzeit aufweist, enthalten auch die weiteren wirkstoffhaltigen Einheiten den zweiten Wirkstoff. In einer besonders bevorzugten Ausführungsform enthalten alle wirkstoffhaltigen Einheiten sowohl den ersten als auch den zweiten Wirkstoff.

### Verteilung der Wirkstoffe

Der erste Wirkstoff ist vorzugsweise in einer Menge von wenigstens 1 mg, weiter bevorzugt wenigstens 10 mg und besonders bevorzugt wenigstens 30 mg in der Arzneiform vorhanden. Die Höchstmenge des ersten Wirkstoffes in der Arzneiform beträgt vorzugsweise 1000 mg, weiter bevorzugt 500 mg, mehr bevorzugt 250 mg und besonders bevorzugt 100 mg. Selbstverständlich hängt die sinnvolle Menge an Wirkstoff in der Arzneiform vom gewählten Wirkstoff ab. Im Rahmen des Arzneiformkonzeptes dieser Erfindung ist die Verteilung der Gesamtmenge an Wirkstoff auf die verschiedenen wirkstoffhaltigen Einheiten wesentlich wichtiger für den Erfolg der Erfindung als die absolute Wirkstoffmenge.

Die bevorzugte Verteilung des ersten Wirkstoffes auf die wirkstoffhaltigen Einheiten hängt von den Resorptionsraten des Wirkstoffes in den verschiedenen Darmabschnitten und der angestrebten Freisetzungscharakteristik ab.

Die Gesamtheit der ersten wirkstoffhaltigen Einheiten enthält zusammen vorzugsweise wenigstens 10 Gew.-%, weiter bevorzugt wenigstens 20 Gew.-% und besonders bevorzugt wenigstens 40 Gew.-% des ersten Wirkstoffes bezogen auf die Gesamtmasse des ersten Wirkstoffes in der Arzneiform. Die Gesamtheit der ersten wirkstoffhaltigen Einheiten enthält vorzugsweise nicht mehr als 70 Gew.-%, weiter bevorzugt nicht mehr als 60 Gew.-% und besonders bevorzugt nicht mehr als 50 Gew.-% des ersten Wirkstoffes bezogen auf die Gesamtmasse des ersten Wirkstoffes in der Arzneiform. Besonders bevorzugt ist ein Anteil von 25 bis 35 Gew.-% des ersten Wirkstoffes in der Gesamtheit der ersten wirkstoffhaltigen Einheiten.

Die Gesamtheit der zweiten wirkstoffhaltigen Einheiten enthält zusammen vorzugsweise wenigstens 10 Gew.-%, weiter bevorzugt wenigstens 20 Gew.-% und besonders bevorzugt wenigstens 40 Gew.-% des ersten Wirkstoffes bezogen auf die Gesamtmasse des ersten Wirkstoffes in der Arzneiform. Die Gesamtheit der zweiten wirkstoffhaltigen Einheiten enthält zusammen vorzugsweise nicht mehr als 70 Gew.-%, weiter bevorzugt nicht mehr als 60 Gew.-% und besonders bevorzugt nicht mehr als 50 Gew.-% des ersten Wirkstoffes bezogen auf die Gesamtmasse des ersten Wirkstoffes in der Arzneiform. Besonders bevorzugt ist ein entsprechender Anteil von 25 bis 35 Gew.-% in der Gesamtheit der zweiten wirkstoffhaltigen Einheiten.

Die Gesamtheit der dritten wirkstoffhaltigen Einheiten enthält zusammen vorzugsweise wenigstens 10 Gew.-%, weiter bevorzugt wenigstens 20 Gew.-% und besonders bevorzugt wenigstens 40 Gew.-% des ersten Wirkstoffes bezogen auf die Gesamtmasse des ersten Wirkstoffes in der Arzneiform. Die Gesamtheit der dritten wirkstoffhaltigen Einheiten enthält vorzugsweise nicht mehr als 70 Gew.-%, weiter bevorzugt nicht mehr als 60 Gew.-% und besonders bevorzugt nicht mehr als 50 Gew.-% des ersten Wirkstoffes bezogen auf die Gesamtmasse des ersten Wirkstoffes in der Arzneiform. Besonders bevorzugt ist ein entsprechender Anteil von 25 bis 35 Gew.-% in der Gesamtheit der dritten wirkstoffhaltigen Einheiten.

Sofern es sich bei dem zweiten Wirkstoff um einen gut wasserlöslichen Wirkstoff handelt, muss dieser nicht unbedingt in kleiner Partikelgröße oder dispergiert in einer Wirkstoffmatrix vorliegen. Sofern es sich allerdings bei dem zweiten Wirkstoff ebenfalls um einen schlecht wasserlöslichen Stoff handelt, wird dieser zweite Wirkstoff vorzugsweise genauso wie der erste Wirkstoff in die wirkstoffhaltigen Einheiten eingebracht, insbesondere ist die Verteilung des zweiten Wirkstoffes auf die wirkstoffhaltigen Einheiten vorzugsweise identisch mit der Verteilung des ersten Wirkstoffes auf die wirkstoffhaltigen Einheiten.

Vorzugsweise werden die Wirkstoffe in etwa zu gleichen Teilen auf die vorhandenen wirkstoffhaltigen Einheiten verteilt. "In etwa" meint dabei, dass Abweichungen in beide Richtungen von etwa 10 Gew.-% noch erfindungsgemäß sind.

Besonders für Wirkstoffe, die eine kurze Halbwertzeit haben, kann es sinnvoll sein, eine Arzneiform mit drei wirkstoffhaltigen Einheiten zu verwenden, von denen eine vorzugsweise im unteren Darmabschnitt den ersten Wirkstoff freisetzt. So kann gewährleistet werden, dass auch viele Stunden nach der Einnahme der Arzneiform weiterer Wirkstoff freigesetzt wird. Eine solche Arzneiform simuliert gleichsam eine dreimal tägliche Einnahme einer schnellfreisetzenden Arzneiform.

Die erfindungsgemäße Arzneiform umfasst vorzugsweise wenigstens eine erste, wenigstens eine zweite und wenigstens eine dritte wirkstoffhaltige Einheit, wobei die zweite und dritte wirkstoffhaltige Einheit so ausgestaltet sind, dass sie den oder die Wirkstoffe über einen längeren Zeitraum freigeben.

Es ist besonders bevorzugt, dass die erste wirkstoffhaltige Einheit keinen magensaftresistenten Überzug aufweist. Die zweite und dritte wirkstoffhaltige Einheit hingegen weisen vorzugsweise einen magensaftresistenten Überzug auf, so dass diese Einheiten im Magen keinen Wirkstoff freisetzen. Alternativ oder zusätzlich zum magensaftresistenten Überzug können die zweite und/oder dritte wirkstoffhaltige Einheit den ersten Wirkstoff in einer Matrix enthalten, die ein magensaftunlösliches Polymer umfasst.

In besonders bevorzugten Ausführungsformen weist die erfindungsgemäße Arzneiform genau zwei Wirkstoffe, nämlich den ersten und den zweiten Wirkstoff auf. In alternativen Ausführungsformen weist die Arzneiform genau einen Wirkstoff auf.

In einer besonders bevorzugten Ausführungsform umfasst die Arzneiform wenigstens eine erste, wenigstens eine zweite und wenigstens eine dritte wirkstoffhaltige Einheit, wobei die erste wirkstoffhaltige Einheit eine schnellfreisetzende Einheit ist, die sich bereits im Magen auflöst. Die zweite und dritte wirkstoffhaltige Einheit sind vorzugsweise magensaftresistent überzogen, so dass sie sich erst im Darm auflösen. Die zweite und die dritte wirkstoffhaltige Einheit weisen vorzugsweise eine Wirkstoffmatrix auf, die ein wasserlösliches Polymer und wenigstens einen ersten und vorzugsweise auch einen zweiten Wirkstoff umfasst.

Mit der erfindungsgemäßen Arzneiform lässt sich eine sequenzielle Wirkstofffreigabe erzielen, wobei eine mehrmals tägliche Einnahme simuliert wird. Dadurch wird die Compliance der Patienten, die mit einer solchen Arzneiform behandelt werden, gegenüber solchen, die mehrmals täglich eine schnellfreisetzende Form einnehmen müssen, verbessert. Durch die Kombination verschiedener kleiner wirkstoffhaltiger Einheiten, die einen oder mehrere Wirkstoffe in unterschiedlichen Abschnitten des Gastrointestinaltraktes freisetzen mit einem in einer Matrix fein dispergierten Wirkstoff kann selbst bei schlecht löslichen Wirkstoffen eine hinreichende Bioverfügbarkeit erzielt werden. Dabei ist die Art des Wirkstoffes für den erfindungsgemäßen Erfolg nicht entscheidend. Tatsächlich kann diese Erfindung auch bei Wirkstoffen eingesetzt werden, die sich sowohl im Magen als auch im Darm schlecht lösen. In diesem Falle wäre es besonders vorteilhaft in allen drei wirkstoffhaltigen Einheiten den Wirkstoff in einer Matrix zu dispergieren, so dass seine Auflösung bereits im Magen unterstützt wird.

Die Arzneiformen dieser Erfindung sind orale Arzneiformen. Das bedeutet, dass sie zur oralen Einnahme bestimmt sind. Es kann sich bei den erfindungsgemäßen Arzneiformen beispielsweise um Kapseln, Tabletten, Granulate, Beutel, Sachets, Pellets oder Sticks handeln.

Aufgrund des einfacheren Aufbaus der ersten wirkstoffhaltigen Einheit, werden in der ersten Einheit weniger Hilfsstoffe benötigt. Daher ist der Gewichtsanteil der ersten wirkstoffhaltigen Einheit an der Arzneiform verhältnismäßig gering. So beträgt der Gewichtsanteil der ersten wirkstoffhaltigen Einheit üblicherweise weniger als 25 Gew.-%, bevorzugt sogar weniger als 20 Gew.-%. Erfindungsgemäß wird der Anteil der ersten wirkstoffhaltigen Einheit vorzugsweise wenigstens 8 Gew.-% und weiter bevorzugt wenigstens 12 Gew.-% betragen.

Der Gewichtsanteil der zweiten wirkstoffhaltigen Einheit an der Arzneiform soll erfindungsgemäß vorzugsweise wenigstens 30 Gew.-%, weiter bevorzugt wenigstens 40 Gew.-% und bevorzugt höchstens 60 Gew.-%, weiter bevorzugt höchstens 50 Gew.-% betragen.

Der Gewichtsanteil der optionalen dritten wirkstoffhaltigen Einheit an der Arzneiform soll erfindungsgemäß vorzugsweise wenigstens 30 Gew.-%, weiter bevorzugt wenigstens 40 Gew.-% und bevorzugt höchstens 60 Gew.-%, weiter bevorzugt höchstens 50 Gew.-% betragen.

Der erfindungsgemäße Nutzen dieser Arzneiform besteht darin, dass die erste wirkstoffhaltige Einheit den ersten Wirkstoff im Magen sofort freisetzt. Der erfindungsgemäße Nutzen dieser Arzneiform kann darin bestehen, dass die zweite wirkstoffhaltige Einheit eine weitere Portion des ersten Wirkstoffes im Dünndarm freisetzt und eine optionale dritte wirkstoffhaltige Einheit im Dickdarm eine weitere Portion des Wirkstoffes freisetzt. Diese Arzneiform ist dann keine Retardarzneiform im herkömmlichen Sinne, denn der Wirkstoff wird, einmal am Ort der Freisetzung angekommen, nicht verlangsamt, sondern sofort freigesetzt. Schlecht lösliche Wirkstoffe lassen sich erfindungsgemäß so freisetzen. Mit einer konventionellen retardierten Form ließe sich dieser Effekt nicht erreichen.

Erfindungsgemäß ist also eine Arzneiform, die den ersten Wirkstoff an mehreren verschiedenen Orten im Gastrointestinaltrakt schnell freisetzt und so die Resorption schlecht löslicher Wirkstoffe ermöglicht.

### Herstellungsverfahren

Die Erfindung betrifft auch ein Herstellungsverfahren für die erfindungsgemäße Arzneiform. Das Verfahren umfasst den Schritt der Fertigung wenigstens einer ersten und wenigstens einer zweiten sowie optional wenigstens einer dritten wirkstoffhaltigen Einheit und das Einbringen wenigstens dieser Einheiten in eine Arzneiform.

Der Schritt der Fertigung jedenfalls der zweiten und dritten wirkstoffhaltigen Einheiten umfasst vorzugsweise die Herstellung einer Wirkstoffmatrix, welche ein Matrixmaterial und den ersten Wirkstoff umfasst. Dieser Schritt umfasst vorzugsweise das Mischen des ersten Wirkstoffes mit dem Matrixmaterial in einem Lösungsmittel und Herstellen eines Granulates.

Die Herstellung des Granulates umfasst vorzugsweise einen Schritt des Trocknens einer Mischung, die Matrixmaterial, Lösungsmittel und ersten Wirkstoff enthält. Das Granulat hat Partikelgrößen von vorzugsweise höchstens 25 µm und vorzugsweise wenigstens 10 µm. Das Lösungsmittel ist vorzugsweise ein Alkanol, insbesondere Methanol, Ethanol, *n*-Propanol oder *iso*-Propanol.

Als Trocknungsverfahren kommen insbesondere Sprühtrocknung, Gefriertrocknung und Vakuumtrocknung in Frage. Sprühtrocknung ist besonders bevorzugt. Das Granulat kann auch durch Aufsprühen einer Wirkstofflösung auf einen Träger aus Matrixmaterial hergestellt werden. Außerdem kann ein Granulierungsschritt vor der Trocknung durchgeführt werden.

Erfindungsgemäß ist auch die Granulatherstellung durch Extrudierung. Das Granulat kann bereits erfindungsgemäß eingesetzte wirkstoffhaltige Einheiten darstellen, insbesondere wirkstoffhaltige Einheiten, die sich im Magen sofort auflösen sollen. Die Eigenschaften der so erhaltenen wirkstoffhaltigen Einheiten hängen dann im Wesentlichen von der Art des Matrixmaterials ab, sofern die Einheiten nicht zusätzlich mit einem Überzug versehen werden.

Optional schließt sich das Anfertigen fester Körper, wie insbesondere Pellets oder Tabletten aus dem erhaltenen Granulat und optionalen Hilfsstoffen an. Die Hilfsstoffe sind insbesondere ausgewählt aus Füllstoffen, Sprengmitteln, Schmiermitteln und Fließmitteln. Besonders bevorzugte Hilfsstoffe sind Laktose, Methylcellulose, Maisstärke, hochdispersem Siliciumdioxid, Talkum und Magnesiumstearat.

Insbesondere dann, wenn das Matrixmaterial keine wesentlichen Anteile an einem magensaftunlöslichen Polymer umfasst, können die festen Körper anschließend mit einem Überzug versehen werden, der vorzugsweise ein magensaftunlösliches Überzugspolymer und einen optionalen Weichmacher umfasst. Dies geschieht vorzugsweise in handelsüblichen Beschichtungsanlagen wie etwa Wirbelschichtanlagen, Coatinganlagen oder Pelletcoatinganlagen. Der Überzug kann die Freisetzungseigenschaften der jeweiligen wirkstoffhaltigen Einheit wesentlich beeinflussen.

Anschließend werden die so erhaltenen wirkstoffhaltigen Einheiten mit optionalen Hilfsstoffen oder weiteren Bestandteilen zu einer erfindungsgemäßen Arzneiform zusammengefügt.

In besonderen Ausführungsformen dieser Erfindung werden die wirkstoffhaltigen Einheiten in eine Hartkapsel eingefüllt. In anderen Ausführungsformen werden die Einheiten in eine Trägermatrix eingebettet. Wieder andere Ausführungsformen sehen vor, dass die wirkstoffhaltigen Einheiten zu einem Körper verpresst werden, so dass ein MUPS erhalten wird. In ebenfalls erfindungsgemäßen Ausführungsformen werden die wirkstoffhaltigen Einheiten in einen Beutel oder in einen Stick gefüllt.

Die vorliegende Erfindung betrifft auch die Verwendung einer erfindungsgemäßen Arzneiform zur Behandlung von Patienten, die einer Behandlung mit schwerlöslichen Wirkstoffen bedürfen. Diese Wirkstoffe sind insbesondere solche, die dreimal täglich verabreicht werden sollen. Solche Wirkstoffe sind insbesondere solche, die oben als erster Wirkstoff beschrieben sind. Bevorzugte Verwendungen dieser Arzneiform sind somit die Behandlung von Schwindel verschiedener Genese.

Wie oben erwähnt kann die erfindungsgemäße Arzneiform eine MUPS-Arzneiform sein. Dabei sind die wirkstoffhaltigen Einheiten vorzugsweise in eine Trägermatrix eingebettet. Eine bevorzugte Rezeptur für eine Trägermatrix, in der die wirkstoffhaltigen Einheiten im Sinne eines MUPS enthalten sein können, kann die folgende Zusammensetzung aufweisen (Angaben in Gew.-%):
Sprengmittel, vorzugsweise mikrokristalline Cellulose: 8 % - 100 %
Bindemittel, vorzugweise Maisstärke: 2 % - 25 %
Fließmittel, vorzugsweise hochdisperses Siliziumdioxid: 0,1 % - 4 %
Schmiermittel, vorzugsweise Magnesiumstearat: 0,1 % - 4 %
Füllstoff, vorzugsweise Kalziumphosphat und/oder Laktose: 3 % - 30 %

Diese Trägermatrix macht in der erfindungsgemäßen MUPS-Arzneiform vorzugsweise wenigstens 30 Gew.-% der Arzneiform aus.

Die erfindungsgemäße Arzneiform weist vorzugsweise einen hohen Gehalt an Sprengmittel (Zerfallsbeschleuniger) auf, um die Auflösung der Arzneiform im Gastrointestinaltrakt zu beschleunigen. Der Gehalt an Sprengmittel kann bis zu 60 Gew.-% betragen. Vorzugsweise liegt der Gehalt an Sprengmittel bei bis zu 50 Gew.-%. Wenigstens 20 Gew.-% sollte der Sprengmittelgehalt betragen, um einen hinreichend raschen Zerfall zu ermöglichen. In bevorzugten Ausführungsformen beträgt der Sprengmittelgehalt wenigstens 30 Gew.-%, besonders bevorzugt wenigstens 40 Gew.-%. Bevorzugte Sprengmittel sind Biopolymere, insbesondere Polysaccharide. Besonders bevorzugt sind Stärken, Stärken und Cellulosen, insbesondere mikrokristalline Cellulose.

Der Gehalt an Matrixpolymer an der Arzneiform wird vorzugsweise 35 Gew.-% nicht überschreiten. Bevorzugt wird der Matrixpolymergehalt höchstens 20 Gew.-% und weiter bevorzugt höchstens 10 Gew.-% betragen. Sofern Matrixpolymer verwendet wird, soll dessen Gehalt vorzugsweise nicht unter 4 Gew.-% und weiter bevorzugt nicht unter 6 Gew.-% betragen.

Die Arzneiform soll insgesamt eine Masse von vorzugsweise höchstens 1000 mg, weiter bevorzugt höchstens 750 mg und mehr bevorzugt höchstens 650 mg aufweisen, damit die Arzneiform leicht eingenommen werden kann. Damit die Handhabbarkeit nicht beeinträchtigt wird, liegt das Mindestgewicht der erfindungsgemäßen Arzneiform allerdings vorzugsweise bei 175 mg, weiter bevorzugt bei 250 mg.

### Beispiele

**Herstellung einer Arzneiform mit Mikrotabletten**

| Inhaltsstoff | Menge pro Arzneiform | Funktion | Monographie |
|---|---|---|---|
| Wirkstoffe | | | |
| | | | |
| Dimenhydrinat | 120.00 mg | 2. Wirkstoff | Ph. Eur. |
| Cinnarizin | 60.00 mg | 1. Wirkstoff | Ph. Eur. |
| Andere Inhaltsstoffe | | | |
| | | | |
| Povidon K30 | 40.00 mg | Matrixpolymer | Ph. Eur. |
| Siliciumdioxid, hochdispers | 2.00 mg | Fließmittel | Ph. Eur. |
| microkrystalline Cellulose | 252.00 mg | Füllstoff, Sprengmittel | Ph. Eur. |
| Pflanzenöl, hydrogeniert | 8.00 mg | Schmiermittel | BP |
| Talk | 20.20 mg | Schmiermittel | Ph. Eur. |
| Magnesiumstearat | 1.00 mg | Schmiermittel | Ph. Eur. |
| Methacrylsäureethylacrylatcopolymer (1:1) | 17.20 mg | Überzugspolymer | Ph. Eur. |
| Methacrylsäuremethylmethacrylatcopolymer (1:2) | 3,44 mg | Überzugspolymer | Ph. Eur. |
| Methacrylsäuremethylmethacrylatecopolymer (1:1) | 13,76 mg | Überzugspolymer | Ph. Eur. |
| Triacetin | 10.32 mg | Weichmacher | Ph. Eur. |
| Isopropanol ¹⁾ | (550.00 mg) | Lösungsmittel | Ph. Eur. |
| Methanol ¹⁾ | (4260.00 mg) | Lösungsmittel | Ph. Eur. |
| gereinigtes Wasser ¹⁾ | (47.60 mg) | Lösungsmittel | Ph. Eur. |
| Gesamtmasse Kapselinhalt | 529.92 mg | | |
| Kapsel | | | |
| Hartkapsel Größe 00eL | | | |

Es wurde eine erfindungsgemäße Arzneiform mit dem ersten Wirkstoff Cinnarizin und dem zweiten Wirkstoff Dimenhydrinat hergestellt. Cinnarizin ist eine schwache Base, die bei einem pH-Wert von 1,1 eine Wasserlöslichkeit von 1,55 mg/ml aufweist. Bei pH 3,0 beträgt die Löslichkeit nur noch etwa 0,05 mg/ml, bei pH 6,5 noch etwa 0,00025 mg/ml. Damit ist Cinnarizin der ideale Wirkstoff, um die Vorteile der vorliegenden Erfindung zu zeigen.

Zunächst wurde eine molekulardisperse Mischung von Cinnarizin mit dem Matrixpolymer Polyvinylpyrrolidon (PVP) hergestellt. Das Cinnarizin wurde also in einer Matrix aus einem wasserlöslichen Polymer dispergiert. In dieser Dispersion liegt der erste Wirkstoff in Partikeln unterhalb der sichtbaren Grenze (< 150 nm) oder teilgelöst vor. Die Dispersion wurde wie folgt hergestellt: Cinnarizin wurde mit PVP mit einem mittleren Molekulargewicht von etwa 40.000 in Aceton versetzt und in einer Sprühtrockenanlage versprüht. Das erhaltene Produkt hatte eine durchschnittliche Korngröße von 10-25 µm und einen Cinnarizingehalt von 12,38 Gew.-% in einer PVP-Matrix.

Als zweite und dritte wirkstoffhaltige Einheit wurden Mikrotabletten mit einem Durchmesser von 1,7 mm und einer durchschnittlichen Höhe von 1,7 mm hergestellt. Diese Mikrotabletten lassen sich sehr gut auf einer Rundlaufpresse mit Mehrfachwerkzeug herstellen, insbesondere wenn die oben aufgeführte Rezeptur eingehalten wird. Es wird dabei das erhaltene Granulat mit den oben genannten Hilfsstoffen zu einer Tablette verpresst.

Die Mikrotabletten wurden wie folgt hergestellt: 161,6 mg des in PVP-Matrix dispergierten Cinnarizins wurden mit 40 mg Dimenhydrinat und einigen Hilfsstoffen zur Fließregulierung sowie zur Verhinderung des Klebens gemischt und auf einer Rundlaufpresse mit Mehrfachwerkzeugen zu Mikrotabletten mit 1,7 mm Durchmesser und 1,7 mm Höhe verpresst.

Die Überzüge wurden in einer Wirbelschichtanlage mit Wurstereinsatz auf die Mikrotabletten aufgebracht.

Zur Herstellung der zweiten wirkstoffhaltigen Einheit wurden die Mikrotabletten mit einem Überzug aus Eudragit® L100-55 mit entsprechender Menge an Weichmacher (Glycerintriacetat) überzogen. Der Überzug ist mindestens 60 Minuten bei einem pH-Wert von 1,2 stabil. Der Überzug wird bei einem pH-Wert von 6,0 bis 6,5 aufgelöst, die Mikrotabletten zerfallen und setzen die Wirkstoffe frei.

Zur Herstellung der dritten wirkstoffhaltigen Einheit wurden die Mikrotabletten mit einem Überzug aus Eudragit® L100 und S100 in einem Massenverhältnis von 2 zu 8 und Glycerintriacetat als Weichmacher überzogen. Die Mikrotabletten haben eine Resistenz von mindestens 60 Minuten im Magen, zerfallen aber noch nicht bei einem pH-Wert von 6,0 bis 6,5 sondern der Überzug löst sich ab einem pH-Wert von 7,0 auf. Auch hier zerfallen anschließend die Multikompartimente und setzen die Wirkstoffe frei.

Außerdem wurde eine erste wirkstoffhaltige Einheit in Form eines Pulvers vorbereitet. Diese erste wirkstoffhaltige Einheit enthielt 20 mg Cinnarizin und 40 mg Dimenhydrinat und Hilfsstoffe.

Darauf wurde eine Kapselmaschine (Zanasi 48E) mit einer Pulverdosierstation und zwei Füllstationen für Mikrotabletten eingerüstet, um die wirkstoffhaltigen Einheiten in Kapseln der Größe 00eL einzufüllen. Diese Kapseln wurden mit jeweils 246 mg der zweiten und dritten wirkstoffhaltigen Einheiten und 80 mg der ersten wirkstoffhaltigen Einheit befüllt.

Somit wurden Arzneiformen mit drei wirkstoffhaltigen Einheiten in einer Kapsel erhalten. Jede wirkstoffhaltige Einheit umfasste 20 mg Cinnarizin und 40 mg Dimenhydrinat.

Die Arzneiform soll 25-45% beider Wirkstoffe innerhalb von 60 Minuten in 0,1 N HCl freigeben. Innerhalb weiterer 60 Minuten bei pH 6,5 sollen insgesamt 60-80% freigegeben werden und nach weiteren 120 Minuten sollen mehr als 80% freigesetzt sein.

### Beschreibung der Abbildungen

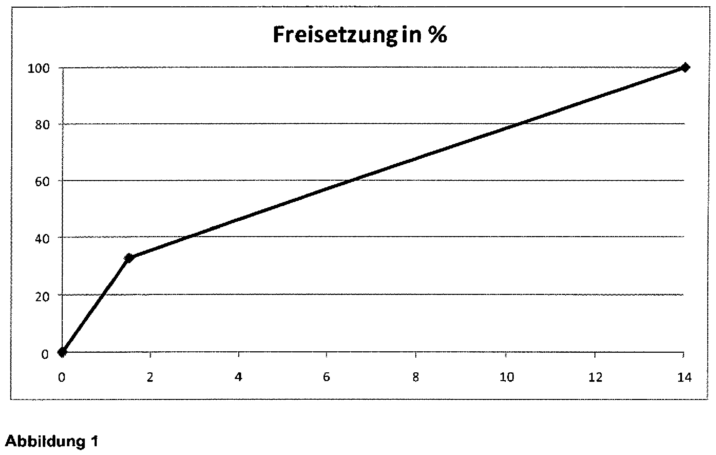

Abbildung 1 zeigt ein theoretisches Freisetzungsprofil einer handelsüblichen Arzneiform, die einen Wirkstoff über einen verlängerten Zeitraum freisetzt.

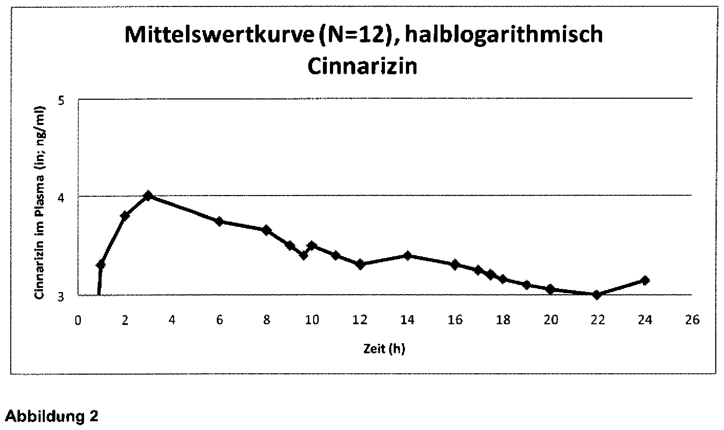

Abbildung 2 zeigt die Plasmaspiegel eines schlecht löslichen Wirkstoffes, die mit einem solchen herkömmlichen Präparat *in vivo* erzielt werden am Beispiel des Cinnarizins. Wie oben diskutiert, ist diese herkömmliche Arzneiform nicht geeignet, eine dreimal tägliche Gabe eines schwach basischen, schlecht wasserlöslichen Arzneistoffes zu simulieren.

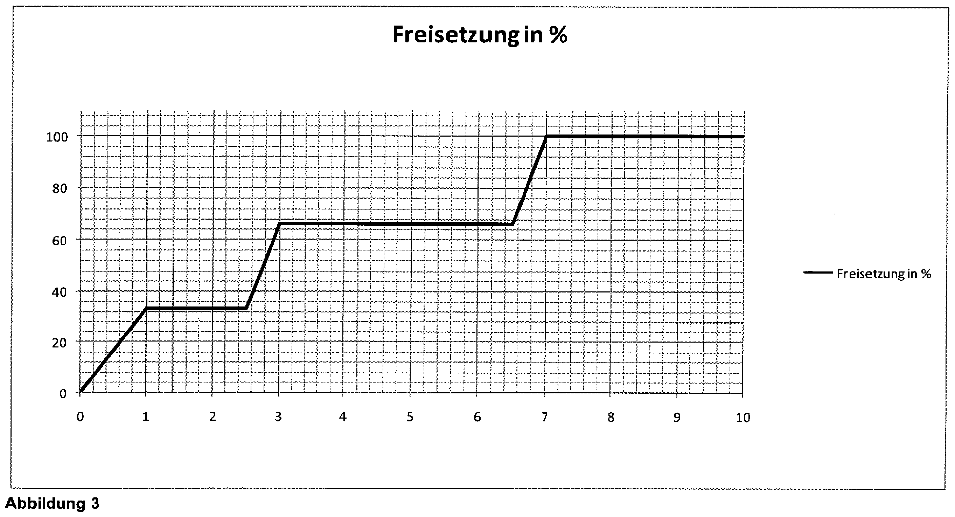

Abbildung 3 zeigt den Freisetzungsverlauf einer erfindungsgemäßen Arzneiform. Die erfindungsgemäße Form setzt den Wirkstoff stufenweise frei, so dass die dreimal tägliche Einnahme simuliert wird. Durch die besondere Ausgestaltung der wirkstoffhaltigen Einheiten wird ein gutes Freisetzungsprofil erzielt. Es ist zu erwarten, dass sich dies auch *in vivo* bestätigen wird.

## Patentansprüche

1. Arzneiform mit
• wenigstens einer ersten wirkstoffhaltigen Einheit und
• wenigstens einer zweiten wirkstoffhaltigen Einheit,
wobei die Einheiten einen ersten Wirkstoff mit wenigstens einer Aminogruppe umfassen, wobei der erste Wirkstoff bei einem pH Wert von 1,1 eine Löslichkeit in Wasser von weniger als 10 mg/ml aufweist, wobei der erste Wirkstoff ausgewählt ist aus der Gruppe der Antihistaminika und/oder Calciumantagonisten,
**dadurch gekennzeichnet,**
**dass** die erste Einheit den ersten Wirkstoff im Magen sofort freisetzt und die zweite Einheit magensaftresistent ausgestaltet ist,
**dass** die zweite wirkstoffhaltige Einheit eine Wirkstoffmatrix enthält, welche ein Matrixpolymer und den ersten Wirkstoff umfasst, wobei der erste Wirkstoff in der Wirkstoffmatrix dispergiert ist
weiterhin **dadurch gekennzeichnet, dass** die Wirkstoffmatrix den ersten Wirkstoff und das Matrixpolymer in einem Masseverhältnis von höchstens 1:1 enthält.

2. Arzneiform nach Anspruch 1, wobei die Wirkstoffmatrix den ersten Wirkstoff und das Matrixpolymer in einem Masseverhältnis von höchstens 1:2 enthält

3. Arzneiform nach Anspruch 1 oder 2, wobei die zweite wirkstoffhaltige Einheit ein Pellet, eine Tablette oder ein Granulat ist.

4. Arzneiform nach wenigstens einem der vorhergehenden Ansprüche, wobei die zweite wirkstoffhaltige Einheit einen magensaftresistenten Überzug aufweist, der ein Überzugspolymer enthält.

5. Arzneiform nach wenigstens einem der vorhergehenden Ansprüche, wobei diese mindestens eine dritte wirkstoffhaltige Einheit aufweist.

6. Arzneiform nach wenigstens einem der vorhergehenden Ansprüche, wobei wenigstens eine der Einheiten einen zweiten Wirkstoff umfasst.

7. Arzneiform nach wenigstens einem der vorhergehenden Ansprüche, wobei das Masseverhältnis von erstem Wirkstoff zu Matrixpolymer in der zweiten wirkstoffhaltigen Einheit höchstens 1:2,3 beträgt.

8. Arzneiform nach wenigstens einem der Ansprüche 5 bis 7, wobei die dritte wirkstoffhaltige Einheit eine Wirkstoffmatrix enthält, welche ein Matrixpolymer und den ersten Wirkstoff umfasst, wobei der erste Wirkstoff in der Wirkstoffmatrix dispergiert ist und das Masseverhältnis von erstem Wirkstoff zu Matrixpolymer in der dritten wirkstoffhaltigen Einheit höchstens 1:1 beträgt.

9. Arzneiform nach wenigstens einem der vorhergehenden Ansprüche, wobei der erste Wirkstoff in der Matrix der zweiten wirkstoffhaltigen Einheit eine mittlere Partikelgröße aufweist, die 1000 nm nicht übersteigt.

10. Arzneiform nach wenigstens einem der vorhergehenden Ansprüche, wobei der erste Wirkstoff in der Matrix der zweiten wirkstoffhaltigen Einheit eine mittlere Partikelgröße aufweist, die nicht mehr als 300 nm beträgt.

11. Verfahren zur Herstellung einer Arzneiform nach einem der vorhergehenden Ansprüche mit den Schritten
a. Herstellen der wirkstoffhaltigen Einheiten,
b. Zusammenfügen der wirkstoffhaltigen Einheiten zu der Arzneiform.

## Claims

1. A dosage form comprising
• at least one first active ingredient-containing unit and
• at least one second active ingredient-containing unit,
wherein the units comprise a first active ingredient with at least one amino group,
wherein the first active ingredient at a pH value of 1.1 has a solubility in water of less than 10 mg/ml, wherein the first active ingredient is selected from the group of antihistamines and/or calcium antagonists,
**characterized in**
**that** the first unit immediately releases the first active ingredient in the stomach and the second unit has a gastric juice resistant design,
**that** the second active ingredient-containing unit contains an active ingredient matrix comprising a matrix polymer and the first active ingredient, wherein the first active ingredient is dispersed in the active ingredient matrix,
furthermore **characterized in that** the active ingredient matrix contains the first active ingredient and the matrix polymer in a mass ratio of at most 1:1.

2. The dosage form according to claim 1, wherein the active ingredient matrix contains the first active ingredient and the matrix polymer in a mass ratio of at most 1:2.

3. The dosage form according to claim 1 or 2, wherein the second active ingredient-containing unit is a pellet, a tablet or a granulate.

4. The dosage form according to at least one of the preceding claims, wherein the second active ingredient-containing unit comprises a gastric juice resistant coating containing a coating polymer.

5. The dosage form according to at least one of the preceding claims, wherein it comprises at least one third active ingredient-containing unit.

6. The dosage form according to at least one of the preceding claims, wherein at least one of the units comprises a second active ingredient.

7. The dosage form according to at least one of the preceding claims, wherein the mass ratio of first active ingredient to matrix polymer in the second active ingredient-containing unit is at most 1:2.3.

8. The dosage form according to at least one of claims 5 to 7, wherein the third active ingredient-containing unit contains an active ingredient matrix comprising a matrix polymer and the first active ingredient, wherein the first active ingredient is dispersed in the active ingredient matrix and the mass ratio of first active ingredient to matrix polymer in the third active ingredient-containing unit is at most 1:1.

9. The dosage form according to at least one of the preceding claims, wherein the first active ingredient in the matrix of the second active ingredient-containing unit has an average particle size which does not exceed 1000 nm.

10. The dosage form according to at least one of the preceding claims, wherein the first active ingredient in the matrix of the second active ingredient-containing unit has an average particle size which is not higher than 300 nm.

11. A method for the production of a dosage form according to one of the preceding claims with the steps of
a. preparing the active ingredient-containing units,
b. assembling the active ingredient-containing units to the dosage form.

## Revendications

1. Forme galénique comportant
- au moins une première unité contenant un principe actif et
- au moins une deuxième unité contenant un principe actif,
dans laquelle les unités comprennent un premier principe actif ayant au moins un groupe amino, dans laquelle le premier principe actif présente à un pH de 1,1, une solubilité dans l'eau de moins de 10 mg/ml, dans laquelle le premier principe actif est choisi dans le groupe des anti-histaminiques et/ou des antagonistes du calcium,
**caractérisée en ce que**
la première unité libère immédiatement le premier principe actif dans l'estomac et la deuxième unité est conçue de façon à résister aux sucs gastriques,
la deuxième unité contenant un principe actif contient une matrice de principe actif qui comprend un polymère de matrice et le premier principe actif, dans laquelle le premier principe actif est dispersé dans la matrice de principe actif,
**caractérisée en outre en ce que** la matrice de principe actif contient le premier principe actif et le polymère de matrice en un rapport en masse d'au plus 1:1.

2. Forme galénique selon la revendication 1, dans laquelle la matrice de principe actif contient le premier principe actif et le polymère de matrice en un rapport en masse d'au plus 1 : 2.

3. Forme galénique selon la revendication 1 ou 2, dans laquelle la deuxième unité contenant un principe actif est une pastille, un comprimé ou un granulé.

4. Forme galénique selon au moins l'une quelconque des revendications précédentes, dans laquelle la deuxième unité contenant un principe actif présente un enrobage résistant aux sucs gastriques qui contient un polymère d'enrobage.

5. Forme galénique selon au moins l'une quelconque des revendications précédentes, dans laquelle celle-ci présente au moins une troisième unité contenant un principe actif.

6. Forme galénique selon au moins l'une quelconque des revendications précédentes, dans laquelle au moins l'une des unités comprend un deuxième principe actif.

7. Forme galénique selon au moins l'une quelconque des revendications précédentes, dans laquelle le rapport en masse du premier principe actif au polymère de matrice dans la deuxième unité contenant un principe actif est au plus de 1 : 2,3.

8. Forme galénique selon au moins l'une quelconque des revendications 5 à 7, dans laquelle la troisième unité contenant un principe actif contient une matrice de principe actif, qui comprend un polymère de matrice et le premier principe actif, dans laquelle le premier principe actif est dispersé dans la matrice de principe actif et le rapport molaire du premier principe actif au polymère de matrice dans la troisième unité contenant un principe actif est au plus de 1 : 1.

9. Forme galénique selon au moins l'une quelconque des revendications précédentes, dans laquelle le premier principe actif dans la matrice de la deuxième unité contenant un principe actif présente une taille moyenne de particule qui ne dépasse pas 1000 nm.

10. Forme galénique selon au moins l'une quelconque des revendications précédentes, dans laquelle le premier principe actif dans la matrice de la deuxième unité contenant un principe actif présente une taille moyenne de particule qui ne dépasse pas 300 nm.

11. Procédé de fabrication d'une forme galénique selon l'une quelconque des revendications précédentes, comportant les étapes consistant à
a. fabriquer les unités contenant un principe actif,
b. assembler les unités contenant un principe actif à la forme galénique.
